# FASCICULE DE BREVET EUROPEEN

(11) **EP 1 333 021 B1**
(45) Date de publication et mention de la délivrance du brevet: **04.10.2017**
(21) Numéro de dépôt: 03290081.3
(22) Date de dépôt: 13.01.2003
(51) Int. Cl.: A61K 8/365, A61Q 19/08

(54) **COMPOSITIONS COMPRENANT DES DÉRIVÉS DE L'ACIDE JASMONIQUE, ET UTILISATION DE CES DÉRIVÉS POUR FAVORISER LA DESQUAMATION**
ZUSAMMENSETZUNGEN ENTHALTEND JASMONSÄUREDERIVATE UND DEREN VERWENDUNG ZUR FÖRDERUNG DER ABSCHUPPUNG DER HAUT
COMPOSITIONS CONTAINING DERIVATIVES OF JASMONIC ACID AND THEIR USE FOR STIMULATING SKIN PEELING

(30) Priorité: 04.02.2002 FR 0201279
(43) Date de publication de la demande: 06.08.2003
(60) Demande divisionnaire: 16200315.6
(73) Titulaire: L'Oréal, 75008 Paris (FR)
(72) Inventeur: Boulle, Christophe, 77400 Lagny S/Marne (FR); Dalko, Maria, 91190 Gif S/Yvette (FR); Leveque, Jean-Luc, 75017 Paris (FR); Simonetti, Lucie, 94300 Vincennes (FR)
(74) Mandataire: Hugodot, Yannick

(56) Documents cités:
- EP-A- 0 989 111
- WO-A-96/00206
- FR-A- 2 718 022
- JP-A- 2001 207 188
- KIYOTA, H. ET AL: "Lipase-catalyzed preparation of both enantiomers of methyl jasmonate" TETRAHEDRON: ASYMMETRY, vol. 12, no. 7, 2001, pages 1035-1038, XP002214851
- DATABASE CA [Online] CHEMICAL ABSTRACTS SERVICE, COLUMBUS, OHIO, US; TAKEUCHI, YASUTOMO ET AL: "Germination inducers containing cucurbic acids for parasitic plants" XP002214852 extrait de STN Database accession no. 131:28910 & JP 11 139907 A (INSTITUTE OF PHYSICAL AND CHEMICAL RESEARCH, JAPAN;NIPPON ZEON CO., LT) 25 mai 1999 (1999-05-25)
- DATABASE CA [Online] CHEMICAL ABSTRACTS SERVICE, COLUMBUS, OHIO, US; 1999, SETO, HIDEHARU ET AL: "Easy preparation of methyl 7-epi-jasmonate and four stereoisomers of methyl cucurbate, and assessment of the stereogenic effect of jasmonate o phytohormonal activities" XP002214853 extrait de STN Database accession no. 130:352121 & BIOSCIENCE, BIOTECHNOLOGY, AND BIOCHEMISTRY (1999), 63(2), 361-367, 1999,
- DATABASE CA [Online] CHEMICAL ABSTRACTS SERVICE, COLUMBUS, OHIO, US; 1999, BLECHERT, S. ET AL: "Structure-activity analyses reveal the existence of two separate groups o active octadecanoids in elicitation of the tendril-coiling response of Bryonia dioica" XP002214854 extrait de STN Database accession no. 130:279324 & PLANTA (1999), 207(3), 470-479, 1999,
- DATABASE CA [Online] CHEMICAL ABSTRACTS SERVICE, COLUMBUS, OHIO, US; 1992, SETO, HIDEHARU ET AL: "Structure-activity relationships of (.+-.)-cucurbic acid analogs on the root growth of rice seedlings and height of young corn plants" XP002214855 extrait de STN Database accession no. 118:118844 & JOURNAL OF PESTICIDE SCIENCE (INTERNATIONAL EDITION) (1992), 17(1), 61-7, 1992,
- PATENT ABSTRACTS OF JAPAN vol. 008, no. 011 (C-205), 18 janvier 1984 (1984-01-18) & JP 58 180410 A (SHISEIDO KK; others: 01), 21 octobre 1983 (1983-10-21)
- DATABASE WPI Section Ch, Week 200204 Derwent Publications Ltd., London, GB; Class D21, AN 2002-029066 XP002346503 & JP 2001 207188 A (POLA CHEM IND INC) 31 juillet 2001 (2001-07-31)

## Description

La présente invention concerne l'utilisation de composés selon la revendication 1 pour favoriser la desquamation de la peau et/ou stimuler le renouvellement épidermique et/ou lutter contre le vieillissement de la peau.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.

Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée stratum corneum.

Le vieillissement cutané résultant de facteurs intrinsèques ou extrinsèques se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies. Certains de ces signes du vieillissement sont plus particulièrement liés au vieillissement intrinsèque ou physiologique, c'est-à-dire au vieillissement "normal" lié à l'âge ou chronobiologique, alors que d'autres sont plus spécifiques du vieillissement extrinsèque, c'est-à-dire du vieillissement provoqué d'une manière générale par l'environnement; il s'agit plus particulièrement du photovieillissement dû à l'exposition au soleil, à la lumière ou à tout autre rayonnement.

L'invention s'intéresse au vieillissement intrinsèque ou physiologique Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique. Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène. On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules mortes situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

La demande JP-A-2001-207188 décrit une composition de parfum contenant du dihydridojasmonate pour supprimer l'activité de la peroxidase et ainsi améliorer la qualité de la peau. La demande WO 96/00206 décrit l'utilisation de l'acide 3-hydroxy 2-pentyl cyclopentaneacétique comme intermédiaire de synthèse pour la préparation de l'acide cis-3-oxo-2-pentyl-1-cyclopentaneacétique.

On constate donc que subsiste le besoin d'agents antivieillissement ayant une action au moins aussi efficace que celle des composés de l'art antérieur, mais ne présentant pas leurs inconvénients.

L'invention a pour but de pallier ces inconvénients de l'art antérieur et de proposer de nouveaux composés susceptibles de favoriser la desquamation de la peau et/ou de stimuler le renouvellement épidermique, dont l'utilisation n'entraînerait pas de picotements, de tiraillements, d'échauffements ou de rougeurs désagréables pour l'utilisateur.

Un autre objet est l'utilisation cosmétique d'un composé de formule (I) selon la revendication 1 pour favoriser la desquamation de la peau, stimuler le renouvellement épidermique, lutter contre les signes du vieillissement cutané, améliorer l'éclat du teint et/ou lisser la peau du visage.

Un autre objet de l'invention est un procédé de traitement cosmétique pour favoriser la desquamation de la peau, stimuler le renouvellement épidermique, lutter contre les signes du vieillissement cutané, améliorer l'éclat du teint et/ou lisser la peau du visage, caractérisé en ce que l'on applique sur la peau une composition cosmétique comprenant un composé de formule (I) telle que définie ci-après.

On a constaté que les composés utilisés selon l'invention présentent une bonne solubilité dans l'eau, ce qui peut faciliter leur mise en oeuvre.

Les composés selon l'invention répondent à la formule (I) suivante : 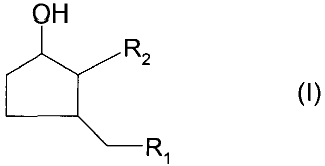 dans laquelle :
- R₁ est un radical -COOH
- R₂ est un radical hydrocarboné, linéaire, ramifié ou cyclique, saturé ou insaturé, ayant 1 à 18 atomes de carbone ;
et ses sels correspondants.

De préférence, le radical R₂ est un radical hydrocarboné, linéaire, ramifié ou cyclique, saturé ou insaturé, ayant 1 à 12 atomes de carbone, et en particulier 1 à 8 atomes de carbone.
Plus particulièrement, R₂ représente un radical hydrocarboné linéaire, saturé ou comportant une insaturation, ayant 2 à 6 atomes de carbone, et notamment un radical -CH₂-CH=CH-C₂H₅ ou un radical -(CH₂)₄-CH₃.

Les sels des composés utilisables selon l'invention sont en particulier choisis parmi les sels de métal alcalin ou alcalino-terreux, ou encore parmi les sels de zinc, de magnésium ou de strontium, d'une amine organique ou les sels d'ammonium quaternaires.
Les sels des composés conformes à l'invention sont en particulier choisis parmi les sels d'un acide minéral ou organique notamment les chlorhydrates, bromhy-drates ou citrates.

Parmi les composés susceptibles d'être employés dans le cadre de l'invention, on peut citer :
- l'acide 3-hydroxy-2-[(2Z)-2-pentenyl]- cyclopentaneacétique,
- l'acide 3-hydroxy-2-pentyl-cyclopentaneacétique.

La quantité de composé de formule (I) utilisable selon l'invention dépend bien évidemment de l'effet recherché et doit être en une quantité efficace pour favoriser la desquamation de la peau et/ou stimuler le renouvellement épidermique et donc lutter contre le vieillissement cutané intrinsèque et/ou extrinsèque.
A titre d'exemple, la quantité de composé de formule (I) utilisable selon l'invention peut aller par exemple de 0,01 à 20% et de préférence de 0,5 à 10%, et notamment de 1 à 5% en poids, par rapport au poids total de la composition.

La composition comprenant les composés selon l'invention, seuls ou en mélange, peut comprendre par ailleurs un milieu physiologiquement acceptable, c'est-à-dire un milieu compatible avec toutes les matières kératiniques telles que la peau, le cuir chevelu, les ongles, les muqueuses, les yeux et les cheveux ou toute autre zone cutanée du corps. Cette composition peut être une composition cosmétique ou pharmaceutique et peut donc comprendre un milieu cosmétiquement ou pharmaceutiquement acceptable.

Le milieu physiologiquement acceptable peut comprendre de l'eau, des solvants organiques tels qu'un alcool en C₁-C₈, notamment l'éthanol, l'isopropanol, le tert-butanol, le n-butanol; un polyol tel que la glycérine ; un glycol comme le butylène glycol, l'isoprène glycol, le propylène glycol, les polyéthylène glycols tels que le PEG-8 ; les éthers de polyol.

La composition peut également comprendre une phase grasse, qui peut comprendre des huiles, des gommes, des cires usuellement utilisées dans le domaine d'application considéré. Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclomé-thicone) et les huiles fluorées (perfluoropolyéthers), les cires d'abeille, de carnauba ou paraffine. On peut ajouter à ces huiles des alcools gras et des acides gras (acide stéarique).

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

La composition peut contenir également des adjuvants habituels dans le domaine considéré, tels que les tensioactifs, les émulsionnants, les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes, les actifs cosmétiques ou pharmaceutiques. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.

Comme tensioactifs susceptibles d'être utilisés, on peut citer par exemple le stéarate de glycérol, le polysorbate 60 et le mélange de PEG-6/PEG-32/Glycol Stéarate vendu sous la dénomination de TefoseR 63 par la société Gattefosse.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.
Parmi les actifs hydrophiles, on peut citer les protéines ou les hydrolysats de protéine, les acides aminés, les polyols, l'urée, l'allantoïne, les sucres et les dérivés de sucre, les vitamines hydrosolubles, les extraits végétaux et les hydroxyacides. Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés. La composition peut associer au moins un composé de formule (I) à d'autres agents actifs, tels que :
- les agents améliorant la repousse et/ou sur le ralentissement de la chute des cheveux, comme par exemple les esters d'acide nicotinique, dont notamment le nicotinate de tocophérol, le nicotinate de benzyle et les nicotinates d'alkyles en C1-C6 comme les nicotinates de méthyle ou d'hexyle, les dérivés de pyrimidine, comme le 2,4-diamino 6-piperidinopyrimidine 3-oxyde ou "Minoxidil", les agents favorisant la repousse des cheveux comme ceux décrits par la demanderesse dans la demande de brevet européen publiée sous le numéro 0648488 ;
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses este rs ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale, marine ou bactérienne.

La composition peut se présenter sous toutes les formes galéniques envisageables.
Notamment, la composition peut avoir la forme de solution aqueuse, alcoolique, hydroalcoolique ou huileuse; de dispersion du type lotion ou sérum; d'émulsion eau-dans-huile, huile-dans-eau ou multiple; de suspension; de microcapsules ou microparticules; de dispersions vésiculaires de type ionique et/ou non ionique; de lotion aqueuse, huileuse ou sous forme de sérum; de capsules, de granulés, de sirops, de comprimés; de mousse, de préparation solide; de composition pour aérosol comprenant également un agent propulseur sous pression.

La composition peut se présenter sous la forme d'une composition pour soins capillaires, notamment un shampooing, une lotion de mise en plis, une lotion traitante, une crème ou un gel coiffant, une composition de teinture, notamment d'oxydation, des lotions restructurantes pour les cheveux, une composition de permanente (notamment une composition pour le premier temps d'une permanente), une lotion ou un gel antichute, un shampooing antiparasitaire.
Elle peut également se présenter sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, comme des lotion de nettoyage, composition de bronzage artificiel); une composition de maquillage du corps ou du visage telle qu'un fond de teint; une composition pour le bain; une composition désodorisante comprenant par exemple un agent bactéricide; une composition après-rasage; une composition épilatoire; une composition contre les piqûres d'insectes; une composition anti-douleur; une composition pour traiter certaines maladies de la peau comme l'eczéma, la rosacée, le psoriasis, les lichens, les prurits sévères.

La composition trouve une application toute particulière comme composition cosmétique destinée au soin de la peau du corps, du visage et/ou du cuir chevelu, notamment pour favoriser la desquamation de la peau, stimuler le renouvellement épidermique, lutter contre les signes du vieillissement cutané, améliorer l'éclat du teint et/ou lisser la peau du visage.
L'invention est illustrée plus en détail dans les exemples suivants.

### Exemple 1 : synthèse de l'acide (1R,2R) 3-hydroxy-2-[(2Z)-2-pentenyl]- cy-clopentaneacétique (+/-) de formule :

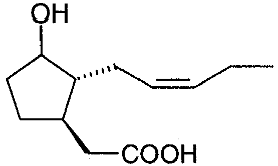

### 1/ étape 1 : synthèse de l'acide jasmonique (+/-) ou acide (1R,2R) 3-oxo-2-[(2Z)-2-pentenyl]- cyclopentaneacétique (+/-)

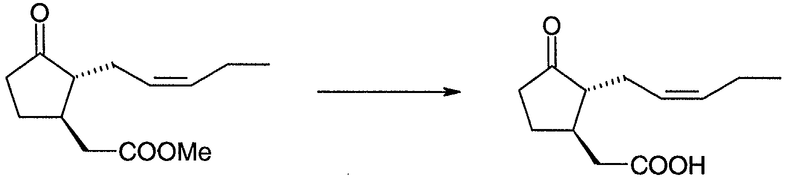

Dans un tricol de 250 ml muni d'un réfrigérant, d'un thermomètre et d'une agitation magnétique, on dissout 15 g (66,9 mmol) de (+/-) jasmonate de méthyle dans 150 ml d'acétone. On additionne lentement 10 ml de solution aqueuse de soude (5,35 g, 133,7 mmol). Le mélange est agité pendant 5 heures à température ambiante. L'acétone est alors évaporée sous vide puis la phase aqueuse résiduelle est lavée par de l'acétate d'éthyle (2 x 30 ml). La phase aqueuse est acidifiée par de l'acide chlorhydrique jusqu'à pH=2, puis extraite par du dichlorométhane (3 x 30 ml).
La phase organique est séchée sur sulfate de sodium, filtrée sur papier filtre puis concentrée. L'huile marron clair obtenue est séchée sous vide.
On obtient 13,6 g d'acide jasmonique (+/-) soit un rendement de 97%.
Le spectre RMN ¹H et le spectre de masse (ionisation négative) sont conformes à la structure attendue.

### 2/ étape 2 : synthèse de l'acide (1R,2R) 3-hydroxy-2-[(2Z)-2-pentenyl]- cyclopen-taneacétique (+/-)

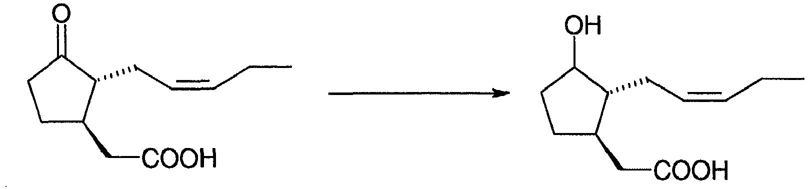

Dans un tricol de 50 ml muni d'un réfrigérant, d'un thermomètre et d'une agitation magnétique, on dissout 1 g (4,8 mmol) d'acide jasmonique (+/-) dans 15 ml d'éthanol absolu. On ajoute 430 mg (11,4 mmol) de borohydrure de sodium NaBH₄. Le mélange est agité pendant 4 heures à 50°C. Une fois la réaction terminée, on ajoute lentement 5 ml d'eau. Le précipité formé est filtré. Le filtrat est acidifié par de l'acide chlorhydrique jusqu'à pH=5 puis extrait par de l'acétate d'éthyle (3 x 30 ml). La phase organique est séchée sur sulfate de sodium, filtrée sur papier filtre puis concentrée. L'huile incolore obtenue est purifiée par chromatographie sur gel de silice (éluant : dichlorométhane/méthanol). L'huile incolore obtenue est séchée sous vide.
On obtient 400 mg du composé recherché, soit un rendement de 40%.
Le spectre RMN ¹H est conforme à la structure attendue.

### Exemple 2 : synthèse du (1R,2R) 2-[(2Z)-2-pentenyl]-3-hydroxy-cyclopenta-neéthanol (+/-) de formule :

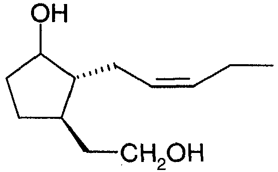

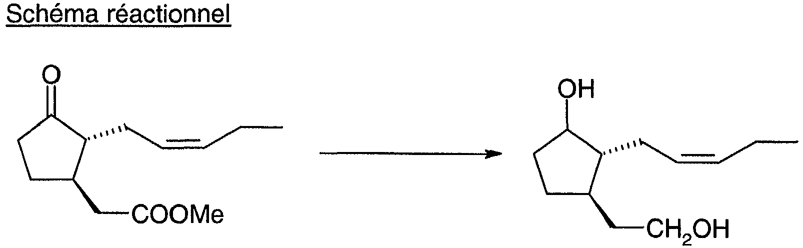

Dans un tricol de 50 ml muni d'un réfrigérant, d'un thermomètre et d'une agitation magnétique, on dissout 1 g (4,5 mmol) de (+/-) jasmonate de méthyle dans 15 ml de tetrahydrofurane. On ajoute 430 mg (11,3 mmol) d'hydrure mixte de lithium et d'aluminium LiAlH₄. Le mélange est agité pendant 4 heures à 50°C. Une fois la réaction terminée, on ajoute lentement 5 ml d'eau. Le précipité formé est filtré et le filtrat est acidifié par de l'acide chlorhydrique jusqu'à pH=5 puis extrait par de l'acétate d'éthyle (3 x 30 ml). La phase organique est séchée sur sulfate de sodium, filtrée sur papier filtre puis concentrée. L'huile incolore obtenue est purifiée par chromatographie sur gel de silice (éluant : dichlorométhane/méthanol). L'huile incolore obtenue est séchée sous vide.
On obtient 550 mg du composé recherché, soit un rendement de 62%.
Le spectre RMN ¹H est conforme à la structure attendue.

### Exemple 3 : Synthèse de l'acide (1R,2R) 3-hydroxy-2-pentyl-cyclopentane-acétique (+/-) de formule :

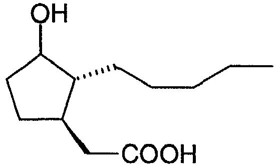

### 1/ étape 1 : synthèse de l'acide dihydrojasmonique (+/-) ou acide (1R,2R) 3-oxo-2-pentyl-cyclopentaneacétique (+/-) de formule :

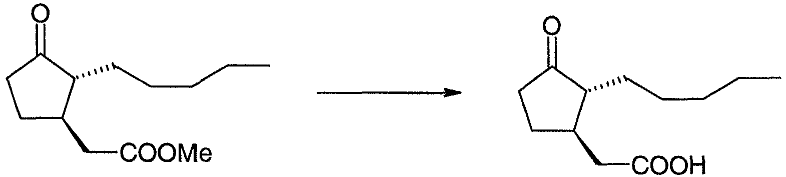

Dans un tricol de 250 ml muni d'un réfrigérant, d'un thermomètre et d'une agitation magnétique, on dissout 5 g (23,6 mmol) de (+/-) dihydrojasmonate de méthyle dans 50 ml d'acétone. On ajoute lentement 10 ml de solution aqueuse de soude (1,76 g, 44 mmol). Le mélange est agité pendant 5 heures à température ambiante. L'acétone est alors distillée sous vide puis la phase aqueuse résiduelle est lavée par de l'acétate d'éthyle (2 x 20 ml). La phase aqueuse est acidifiée par de l'acide chlorhydrique jusqu'à pH=2. Cette phase est alors extraite par du dichlorométhane (2 x 30 ml). La phase organique est séchée sur sulfate de sodium, filtrée sur papier filtre puis concentrée. L'huile obtenue est purifiée par chromatographie sur gel de silice (éluant : dichlorométhane / méthanol). L'huile obtenue est séchée sous vide.
On obtient 1,7 g du composé recherché, soit un rendement de 36%.
Le spectre RMN ¹H est conforme à la structure attendue.

### 2/ étape 2 : synthèse de l'acide (1R,2R) 3-hydroxy-2-pentyl-cyclopentane-acétique (+/-)

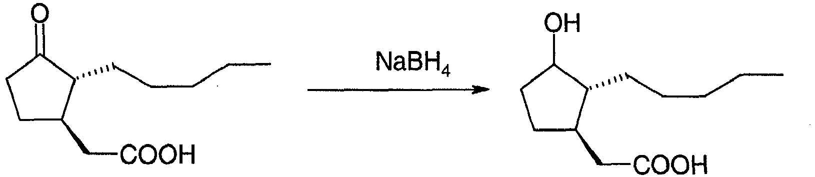

On prépare ce composé de manière similaire à celle décrite à l'étape 2 de l'exemple 1, en faisant réagir de l'acide dihydrojasmonique avec du borohydrure de sodium NaBH₄, dans l'éthanol, pendant 4 heures à 50°C.
On obtient le composé recherché avec un rendement de 45%.
Le spectre RMN ¹H est conforme à la structure attendue.

### Exemple 4 : synthèse du (1R,2R) 2-pentyl-3-hydroxy-cyclopentaneéthanol (+/-) de formule :

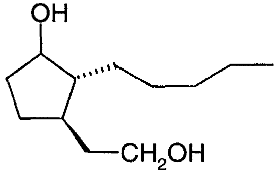

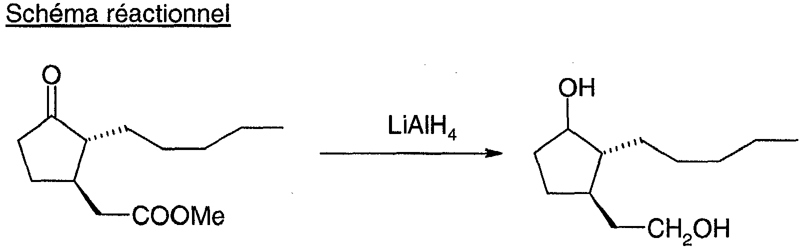

On prépare ce composé de manière similaire à celle décrite à l'exemple 2, en faisant réagir du dihydrojasmonate de méthyle dans le tetrahydrofurane avec l'hydrure mixte de lithium et d'aluminium, pendant 4 heures à 50°C.
On obtient le composé recherché avec un rendement de 68%.
Le spectre RMN ¹H est conforme à la structure attendue.

### Exemple 5 : tests d'activité

On étudie le pouvoir kératolytique de plusieurs composés selon l'invention. Ce test consiste en un comptage de cornéocytes libérés après incubation des lots de stratum corneum isolé en présence des composés testés.

On utilise du stratum corneum isolé par trypsine/chaleur à partir de plasties de chirurgie. On utilise plusieurs échantillons de stratum corneum différents. Des disques de 4 mm de diamètre sont coupés à l'emporte pièce et disposés au fond d'une boite de 96 puits.

### Test 1

On prépare une solution à 1% en poids de composé de l'exemple 3 dans un tampon PBS supplémenté à 0,1% en Triton X100. Le pH de la solution est réajusté à 7,4.

On ajoute dans chaque puits 50 microlitres de solution à tester ou de solution témoin (tampon PBS supplémenté à 0,1% en TritonX100). On laisse incuber à 37°C sous agitation pendant 24 heures.

On prélève alors 10 microlitres de solution, que l'on dispose en cellule de Mallassez. Les cornéocytes libérés sont comptés sous microscope.

On obtient les résultats suivants, exprimés en nombre de cornéocytes libérés par microlitre, moyennés pour trois essais. Les fragments de cornéocytes ne sont pas comptés.

| | Echantillon 1 * | Echantillon 2 * | Echantillon 3 * | Moyenne |
|---|---|---|---|---|
| Exemple 3 | 28 ± 9 | 56 ± 7 | 101 ± 11 | 61 ± 33 |
| Témoin | 4 ± 4 | 8 ± 3 | 17 ± 8 | 9 ± 8 |

| | | | | |
|---|---|---|---|---|
| * moyenne sur trois essais | | | | |

Le nombre de cornéocytes libérés après incubation du stratum corneum isolé avec le composé selon l'invention est très supérieur au nombre libéré en présence du tampon seul.

### Test 2

On prépare une solution à 1% en poids de composé de l'exemple 1 ou d'acide jasmonique (comparatif) dans un tampon PBS supplémenté à 0,1% en Triton X100. Le pH des solutions est réajusté à 7,4.

On ajoute dans chaque puits 50 microlitres de solution à tester ou de solution témoin (tampon PBS supplémenté à 0,1% en TritonX100). On laisse incuber à 37°C sous agitation pendant 24 heures.

On prélève alors 10 microlitres de solution, que l'on dispose en cellule de Mallassez. Les cornéocytes libérés sont comptés sous microscope.

On obtient les résultats suivants, exprimés en nombre de cornéocytes libérés par microlitre, moyennés pour trois essais. Les fragments de cornéocytes ne sont pas comptés.

| | Moyenne (3 essais par échantillon, 3 échantillons différents) |
|---|---|
| Exemple 1 | 26 ± 7 |
| Acide jasmonique | 15 ± 5 |
| Témoin | 9 ± 3 |

Les résultats nous indiquent que le composé de l'exemple 1 permet une meilleure libération de cornéocytes que l'acide jasmonique par rapport au témoin.

### Test 3

On prépare une solution à 2% en poids de composé de l'exemple 3, ou d'acide 2-hydroxy-4-octanoyl-benzoïque (comparatif), dans une solution aqueuse comprenant 50% en poids de polyéthylène glycol (PEG 8) et 30% en poids d'éthanol.

On ajoute dans chaque puits 50 microlitres de solution à tester ou de solution témoin. On laisse incuber à 37°C sous agitation pendant 24 heures.

On prélève alors 10 microlitres de solution, que l'on dispose en cellule de Mallassez. Les cornéocytes libérés sont comptés sous microscope.

On obtient les résultats suivants, exprimés en nombre de cornéocytes libérés par microlitre, moyennés pour trois essais. Les fragments de cornéocytes ne sont pas comptés.

| | Echantillon 1 * | Echantillon 2 * | Moyenne |
|---|---|---|---|
| Exemple 3 | 2 ± 3 | 3 ± 1 | 2,5 ± 2 |
| Acide 2-hydroxy-4-octanoyl-benzoïque | 3 ± 3 | 4 ± 3 | 3,5 ± 3 |
| Témoin | 1 ± 1 | 1 ± 1 | 1 ± 1 |

| | | | |
|---|---|---|---|
| * moyenne sur trois essais | | | |

### Exemple 6

On prépare une émulsion comprenant (% en poids) :
- composé de l'exemple 1 1 %
- isostéarate de propylène glycol 13%
- polyéthylène glycol (8 OE) 5%
- propylène glycol 3%
- pentylène glycol 3%
- stéarate de glycéryle et stéarate de polyéthylène glycol (100 OE) 5%
- mono-stéarate de sorbitane oxyéthyléné (20 OE) 0,5%
- alcool cétylique oxyéthyléné (20 OE) oxypropyléné (5 OP) 1 %
- gélifiant 0,5%
- benzoates d'alkyle en C₁₂₋₁₅ 4%
- éthanol 3%
- hydroxyde de sodium 0,12%
- conservateurs qs
- eau qsp 100%

## Revendications

1. Utilisation cosmétique d'au moins un composé de formule (I) : 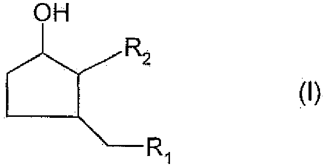 dans laquelle :
- R₁ est un radical -COOH
- R₂ est un radical hydrocarboné, linéaire, ramifié ou cyclique, saturé ou insaturé, ayant 1 à 18 atomes de carbone ;
et ses sels correspondants,
, pour favoriser la desquamation de la peau, stimuler le renouvellement épidermique, lutter contre les signes du vieillissement cutané, améliorer l'éclat du teint et/ou lisser la peau du visage.

2. Utilisation selon la revendication précédente, dans laquelle le radical R₂ est un radical hydrocarboné, linéaire, ramifié ou cyclique, saturé ou insaturé, ayant 1 à 12 atomes de carbone, et en particulier 1 à 8 atomes de carbone.

3. Utilisation selon l'une des revendications précédentes, dans laquelle le radical R₂ représente un radical hydrocarboné linéaire, saturé ou comportant une insaturation, ayant 2 à 6 atomes de carbone, et notamment un radical -CH₂-CH=CH-C₂H₅ ou un radical -(CH₂)₄-CH₃.

4. Utilisation selon l'une des revendications précédentes, dans laquelle le composé de formule (I) est choisi parmi :
- l'acide 3-hydroxy-2-[(2Z)-2-pentenyl]- cyclopentaneacétique,
- l'acide 3-hydroxy-2-pentyl-cyclopentaneacétique.

5. Utilisation selon l'une des revendications précédentes, dans laquelle le composé de formule (I) est l'acide 3-hydroxy-2-pentyl-cyclopentaneacétique.

6. Utilisation selon l'une des revendications précédentes, dans laquelle le sel du composé de formule (I) est choisi parmi les sels de métal alcalin ou alcalino-terreux, les sels de zinc, de magnésium ou de strontium, les sels d'une amine organique ou les sels d'ammonium quaternaires.

7. Utilisation selon l'une des revendications précédentes, dans laquelle le sel du composé de formule (I) est choisi parmi les sels de métal alcalin.

8. Procédé de traitement cosmétique non thérapeutique pour favoriser la desquamation de la peau, stimuler le renouvellement épidermique, lutter contre les signes du vieillissement cutané, améliorer l'éclat du teint et/ou lisser la peau du visage, **caractérisé en ce que** l'on applique sur la peau une composition cosmétique comprenant un composé de formule (I) tel que défini selon l'une des revendications 1 à 7.

9. Procédé de traitement cosmétique selon la revendication précédente, **caractérisé en ce que** le composé de formule (I) est présent dans la composition en une quantité allant de 0,01 à 20%, de préférence de 0,5 à 10%, et notamment de 1 à 5% en poids, par rapport au poids total de la composition.

## Patentansprüche

1. Kosmetische Verwendung mindestens einer Verbindung der Formel (I) : 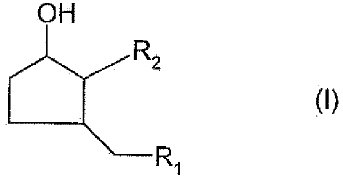 in der:
- R₁ für einen -COOH-Rest steht,
- R₂ für einen gesättigten oder ungesättigten, linearen, verzweigten oder cyclischen Kohlenwasserstoffrest mit 1 bis 18 Kohlenstoffatomen steht;
und der entsprechenden Salze davon
zur Förderung der Desquamation der Haut, Stimulierung der Epidermiserneuerung, Bekämpfung der Anzeichen von Hautalterung, Verbesserung des Strahlens des Teints und/oder Glättung der Gesichtshaut.

2. Verwendung nach dem vorhergehenden Anspruch, wobei der Rest R₂ für einen gesättigten oder ungesättigten, linearen, verzweigten oder cyclischen Kohlenwasserstoffrest mit 1 bis 12 Kohlenstoffatomen und insbesondere 1 bis 8 Kohlenstoffatomen steht.

3. Verwendung nach einem der vorhergehenden Ansprüche, wobei der Rest R₂ für einen gesättigten oder eine Ungesättigtheit enthaltenden, linearen Kohlenwasserstoffrest mit 2 bis 6 Kohlenstoffatomen und insbesondere einen -CH₂-CH=CH-C₂H₅-Rest oder einen -(CH₂)₄-CH₃-Rest steht.

4. Verwendung nach einem der vorhergehenden Ansprüche, wobei die Verbindung der Formel (I) aus
- 3-Hydroxy-2-[(2Z)-2-pentenyl]cyclopentanessig-säure und
- 3-Hydroxy-2-pentylcyclopentanessigsäure ausgewählt ist.

5. Verwendung nach einem der vorhergehenden Ansprüche, wobei sich bei der Verbindung der Formel (I) um 3-Hydroxy-2-pentylcyclopentanessig-säure handelt.

6. Verwendung nach einem der vorhergehenden Ansprüche, wobei das Salz der Verbindung der Formel (I) aus Alkali- oder Erdalkalimetallsalzen, Zink-, Magnesium- oder Strontiumsalzen, Salzen eines organischen Amins oder quaternären Ammoniumsalzen ausgewählt ist.

7. Verwendung nach einem der vorhergehenden Ansprüche, wobei das Salz der Verbindung der Formel (I) aus Alkalimetallsalzen ausgewählt ist.

8. Verfahren zur nichttherapeutischen kosmetischen Behandlung zur Förderung der Desquamation der Haut, Stimulierung der Epidermiserneuerung, Bekämpfung der Anzeichen von Hautalterung, Verbesserung des Strahlens des Teints und/oder Glättung der Gesichtshaut, **dadurch gekennzeichnet, dass** man auf die Haut eine kosmetische Zusammensetzung, die eine Verbindung der Formel (I) gemäß einem der Ansprüche 1 bis 7 umfasst, aufbringt.

9. Verfahren zur kosmetischen Behandlung nach dem vorhergehenden Anspruch, **dadurch gekennzeichnet, dass** die Verbindung der Formel (I) in der Zusammensetzung in einer Menge im Bereich von 0,01 bis 20 Gew.-%, vorzugsweise 0,5 bis 10 Gew.-% und insbesondere 1 bis 5 Gew.-%, bezogen auf das Gesamtgewicht der Zusammensetzung, vorliegt.

## Claims

1. Cosmetic use of at least one compound of formula (I) : 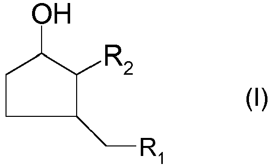 in which:
- R₁ is a -COOH radical
- R₂ is a linear, branched or cyclic, saturated or unsaturated hydrocarbon-based radical containing 1 to 18 carbon atoms;
and the corresponding salts thereof,
for promoting desquamation of the skin, stimulating epidermal renewal, combating the signs of ageing of the skin, improving the radiance of the complexion and/or smoothing out facial skin.

2. Use according to the preceding claim, in which the radical R₂ is a linear, branched or cyclic, saturated or unsaturated hydrocarbon-based radical containing 1 to 12 carbon atoms, and in particular 1 to 8 carbon atoms.

3. Use according to either of the preceding claims, in which the radical R₂ represents a linear hydrocarbon-based radical, which is saturated or comprising an unsaturation, containing 2 to 6 carbon atoms, and especially a -CH₂-CH=CH-C₂H₅ radical or a -(CH₂)₄-CH₃ radical.

4. Use according to one of the preceding claims, in which the compound of formula (I) is chosen from:
- 3-hydroxy-2-[(2Z)-2-pentenyl]cyclopentaneacetic acid,
- 3-hydroxy-2-pentylcyclopentaneacetic acid.

5. Use according to one of the preceding claims, in which the compound of formula (I) is 3-hydroxy-2-pentylcyclopentaneacetic acid.

6. Use according to one of the preceding claims, in which the salt of the compound of formula (I) is chosen from alkali metal or alkaline-earth metal salts, zinc, magnesium or strontium salts, salts of an organic amine or quaternary ammonium salts.

7. Use according to one of the preceding claims, in which the salt of the compound of formula (I) is chosen from the alkali metal salts.

8. Non-therapeutic cosmetic treatment process for promoting desquamation of the skin, stimulating epidermal renewal, combating the signs of ageing of the skin, improving the radiance of the complexion and/or smoothing out facial skin, **characterized in that** a cosmetic composition comprising a compound of formula (I) as defined according to one of Claims 1 to 7 is applied to the skin.

9. Cosmetic treatment process according to the preceding claim, **characterized in that** the compound of formula (I) is present in the composition in an amount ranging from 0.01% to 20%, preferably from 0.5% to 10% and especially from 1% to 5% by weight relative to the total weight of the composition.
